# EUROPEAN PATENT SPECIFICATION

(11) **EP 2 830 641 B1**
(45) Date of publication and mention of the grant of the patent: **08.08.2018**
(21) Application number: 13709193.0
(22) Date of filing: 13.03.2013
(51) Int. Cl.: A61K 36/84, A61P 25/22, A61P 25/24, A61K 31/192

(54) **NEW VALERIAN EXTRACTS AND USES THEREOF**
NEUE EXTRAKTE AUS BALDRIAN SOWIE DEREN VERWENDUNG
NOUVEAUX EXTRAITS DE VALERIANE ET LEURS UTILISATIONS

(30) Priority: 30.03.2012 EP 12002341
(43) Date of publication of application: 04.02.2015
(73) Proprietor: Brattström, Axel, 39108 Magdeburg (DE)
(72) Inventor: Brattström, Axel, 39108 Magdeburg (DE)
(74) Representative: Kasche, André
(86) International application number: PCT/EP2013/055087
(87) International publication number: WO 2013/143843

(56) References cited:
- WO-A1-01/07063
- WO-A1-02/092113

## Description

The present invention relates to a valerian extract for use in the treatment and prophylaxis of GABA-related disorders selected from the group consisting of anxiety, depression, restlessness and insomnia, wherein the ratio of valerenic acid (VA) to acetoxyvalerenic acid (AVA) in the extract is at least 2:1 by weight. The invention also teaches pharmaceutical compositions comprising such extracts as well as methods for preparing these extracts.

### Background of the invention

Valerian is one of the most commonly used herbal remedies for the treatment of insomnia and anxiety. Valerenic acid (VA) is one of the many active compounds in valerian. The anti-anxiety potential of VA has been verified by *in-vitro* experiments on the gamma-aminobutyric acid type A receptor (GABA_{A}) as well as by *in-vivo* experiments in mice and rats. The VA action on the GABA_{A} receptors *in-vitro* is preferentially mediated by the β3 subunits as confirmed by *in-vivo* experiments that demonstrate anxiolytic activity, because a point mutation in the β3 subunit abolished the anxiolytic response to VA but not to diazepam.

GABA is the major inhibitory neurotransmitter in the brain. It is essential for the overall balance of neuronal excitation and inhibition and, thus, vital for the normal brain function. An imbalance may cause GABA-related disorders like anxiety, depression, restlessness, sedation and insomnia. GABA_{A} receptors are the site of action for a variety of pharmacologically important agents, such as benzodiazepines, barbiturates, neuroactive steroids, anesthetics, and anti-convulsants.

It has been shown that valerian extracts (VE) allosterically modulate GABA_{A} receptors and that this action is related to VA. Supposedly, the GABA_{A} receptors containing β2 or β3 subunits are responsible for this action. Derivatives of the VA, i.e. acetoxyvalerenic acid (AVA) or hydroxyvalerenic acid (HVA) do not allosterically modulate GABA_{A} receptors. However AVA at higher concentrations can block the open GABA_{A} channel like HVA and, moreover, HVA has been shown to displace ³H valerenic acid from brain membrane binding sites, thus, indicating that both VA derivatives may act on the identical binding site even though they do fail in allosteric modulation.

Today, VA and its derivatives are the commonly used markers for the qualitative and quantitative analysis of valerian roots, rhizomes as well as VEs. Valerian products are usually standardized to a percent of VA, defined as the amount of VA and AVA and, sometimes, HVA is also included. Whilst VA is stable in the extract, AVA is converted into HVA with time and depending on storage conditions. Most of the presently available valerian extracts used for medical treatment feature a ratio of VA to AVA of about 1:3 to 1:1 by weight.

It is the objective of the present invention to provide improved valerian extracts and pharmaceutical compositions based on these valerian extracts. It is a further preferred objective to provide improved medicaments for use in the treatment and prophylaxis of GABA-related disorders, in particular the GABA_{A}-related disorders anxiety, depression, restlessness, and insomnia.

The above objects are solved by a valerian extract for use in the treatment and prophylaxis of GABA-related disorders selected from the group consisting of anxiety, depression, restlessness and insomnia, wherein the ratio of valerenic acid (VA) to acetoxyvalerenic acid (AVA) in the extract is at least 2:1 by weight.

It was surprisingly found that valerian extracts featuring a ratio of VA to AVA of at least 2:1 by weight target and affect the GABA receptor more efficiently than the extracts commonly used in the art, which regularly feature a ratio of 1:3 to 1:1 by weight. The ratio of the inventive extract further has the advantage that the overall content of valerenic acid and valerenic acid derivatives can be reduced significantly, thereby avoiding typical side effects that go along with intoxication.

In a more preferred embodiment the extract for use according to the invention is one, wherein the ratio of valerenic acid (VA) to acetoxyvalerenic acid (AVA) in the extract is at least 3:1, preferably at least 4:1, more preferably at least 10:1, most probably at least about 12:1 by weight.

The term "valerian extract" as used herein is defined as any extraction product of valerian roots and rhizomes that comprises at least valerian sesquiterpenes (at least VA and AVA) as well as detectable amounts of at least one, preferably more of flavonoids, triterpenes, lignans, alkaloids and amino acids, preferably valerian sesquiterpenes and detectable amounts of flavonoids, triterpenes, lignans, alkaloids and amino acids.

Preferably, the valerian extract is the result of the extraction of valerian roots and/or rhizomes with lipophylic solvents, preferably with at least partially lipophilic solvents, more preferably a solvent selected from the group consisting of petrolether, ethyl acetate and/or aqueous alcohols, so called hydro-alcohols, preferably aqueous methanol or ethanol, more preferably aqueous ethanol. The term lipophylic extract, as used herein, refers to a valerian extract produced with a lipophilic solvent or an at least partially lipophilic solvent, e.g. the solvents mentioned above, that is suited for solvating valerenic acid. A lipophilic or partially lipophilic solvent for practicing the present invention excludes pure water but includes any solvents and solvent mixtures that substantially solvate and extract valerenic acid. The solvent can be more lipophilic, e.g. petrolether, or less lipophilic, e.g. hydroalcohols. For example, an about 70 % aqueous ethanol or an about 50 % aqueous methanol solvent are preferred for extracting valerenic acid. However, a more polar, i.e. hydrophilic solvent, e.g. 30 % hydroalcohol is unlikely to extract enough valerenic acid to produce a valerian extract for practicing the present invention. The preparation of valerenic acid-containing valerian extracts has been abundantly published in the art, e.g. in the European Pharmacopoeia Supplement 5.7. *Valerian* dry hydroalcoholic extract (1898). Today, valerian extracts for medical use are regularly prepared by extraction with hydro-alcohols with an alcohol content between 40 - 80%. The skilled person can readily select suitable solvents to preparing the valerian extract for use in the present invention.

The inventors have conducted a broad screening of valerian plants and so far have identified one valerian plant which naturally produces valerenic acid in a much higher content than its derivatives. This single plant was multiplied to allow for the production of extract amounts of VE which permit *in-vivo* experimental validation of its pharmaceutical utility and advantages.

Alternatively, valerian extracts for use in the present invention can be prepared either by reducing the content of AVA, for example by fractionation of a standard extract, or by artificially increasing the valerenic acid content, for example by spiking a standard extract with synthetic, isolated and/or concentrated VA.

The extract for use according to the present invention is particularly suited to modulate GABA_{A} receptors, more preferably GABA_{A} receptors containing β2 or β3 subunits. In a preferred embodiment the invention relates to the inventive valerian extract for use in the treatment and prophylaxis of GABA_{A}-related disorders, preferably disorders related to GABA_{A} receptors containing β2 or β3 subunits.

In a further aspect, the present invention is directed to the use of the above valerian extract for the preparation of a medicament for use in the treatment and prophylaxis of GABA_{A}-related disorders, preferably selected from the group consisting of anxiety, depression, restlessness and insomnia.

Another aspect of the present invention relates to a pharmaceutical composition comprising a valerian extract of the invention. Pharmaceutical compositions of the present invention typically comprise a therapeutically or prophylactically effective amount of a valerian extract according to the invention and optionally auxiliary substances such as pharmaceutically acceptable excipient(s) and/or carrier(s). Also, the pharmaceutical composition may comprise further extract components and/or medically active agent

More preferably, the pharmaceutical composition also comprises at least one extract selected from the group of extracts produced from balm [melissa], hop and passion flower. In a preferred embodiment, the pharmaceutical composition further comprises extract(s) produced from balm [melissa], hop and passion flower.

A further aspect of the present invention is a method for preparing a valerian extract comprising the steps of:
a) selecting valerian roots and/or rhizomes, wherein the ratio of valerenic acid (VA) to acetoxyvalerenic acid (AVA) is at least 2:1 or at least 3:1, preferably at least 4:1, more preferably at least 10:1, most probably at least about 12:1 by weight.
b) extracting the valerenic acid (VA) and acetoxyvalerenic acid (AVA) from the roots and/or rhizomes using a suitable solvent,
c) optionally further processing the raw extract to make it suitable for human administration.

Preferably, the suitable solvent is lipophilic, more preferably at least partially lipophilic, most preferably a solvent as mentioned above. The skilled person can routinely select valerian roots and/or rhizomes having the required ratio of VA to AVA. Examples of such valerian plants are mentioned above and in the examples below. Also, the skilled person is aware of suitable solvents for valerian extraction, e.g. petrolether, ethyl acetate and hydro-alcohols.

The lipophylic extract can and most often is further processed for making it suitable for human administration, i.e. it is quantified, e.g. standardized for VA, AVA and other valerian components, and it is formulated for the therapeutically or prophylactically effective dosage, for storage stability and the particular route of administration, preferably oral administration.

A further preferred method for preparing a valerian extract of the invention comprises the steps of:
a) providing valerian roots and/or rhizomes,
b) extracting the valerenic acid (VA) and acetoxyvalerenic acid (AVA) from the roots and/or rhizomes using a suitable solvent,
c) adapting the content of valerenic acid (VA) and acetoxyvalerenic acid (AVA) in the extract so that the ratio of valerenic acid (VA) to acetoxyvalerenic acid (AVA) is at least 2:1 or at least 3:1, preferably at least 4:1, more preferably at least 10:1, most probably at least about 12:1 by weight and
d) optionally further processing the extract to make it suitable for human administration.

Preferably, the suitable solvent is lipophilic, more preferably at least partially lipophilic, most preferably a solvent as mentioned above. For example, the content of VA and AVA in the extract can be adapted to the desired ratio by the addition of VA and/or the reduction of AVA. For example, synthetic, isolated and/or concentrated VA may be added to the extract until the desired ratio of A to VA of the invention is reached. The preferred administration mode is oral. Preferably, the valerian roots and/or rhizomes are from *Valeriana officinalis* L.

Also described herein is a method for the treatment and prophylaxis of GABA_{A}-related disorders, comprising the step of administering a valerian extract to a patient in need thereof, wherein the ratio of valerenic acid (VA) to acetoxyvalerenic acid (AVA) in the extract is at least 2:1 or at least 3:1, preferably at least 4:1, more preferably at least 10:1, most probably at least about 12:1 by weight.

For therapeutic or prophylactic use the extracts of the invention may be administered in any conventional dosage form in any conventional manner. Routes of administration include, but are not limited to, intravenously, intraperitoneally, intramuscularly, subcutaneously, by infusion, sublingually, transdermally, orally, rectally and topically. The preferred mode of administration is oral, preferably in a solid or liquid dosage form.

The extracts may be administered alone or in combination with adjuvants that enhance stability of the extracted components, facilitate administration of pharmaceutical compositions containing them, provide increased dissolution or dispersion, support bioavailability, provide adjunct therapy, and the like, including other active ingredients. Advantageously such combination therapies utilize lower dosages of the conventional therapeutics, thus avoiding possible toxicity and adverse side effects incurred when those agents are used as monotherapies.

As mentioned above, dosage forms of the compounds described herein include pharmaceutically acceptable carriers and adjuvants known to those of ordinary skill in the art. These carriers and adjuvants include, for example, ion exchangers, alumina, aluminium stearate, lecithin, serum proteins, buffer substances, water, salts or electrolytes and cellulose-based substances. Also, maltodextrin can be used to produce a free-flowing dry extract which is particularly suitable for further processing into tablets and capsules. Preferred dosage forms include, tablet, capsule, caplet, liquid, solution, suspension, emulsion, lozenges, syrup, reconstitutable powder, granule, suppository and transdermal patch. Controlled release dosage forms with or without immediate release portions are also envisaged. Methods for preparing such dosage forms are known (see, for example, H. C. Ansel and N. G. Popovish, Pharmaceutical Dosage Forms and Drug Delivery Systems, 5th ed., Lea and Febiger (1990)). Dosage levels and requirements are well-recognized in the art and may be selected by those of ordinary skill in the art from available methods and techniques suitable for a particular patient. In preferred embodiments, the administered valerian extract amount contains 0.2 - 0.5 mg/kg body weight VA whilst the AVA content should be kept as small as possible. Although one dose per day may be sufficient, up to 5 doses per day may be given. For oral doses, up to 200 mg/day may be required. As the person skilled in the art will appreciate, lower or higher doses may be required depending on particular factors. For instance, specific doses and treatment regimens will depend on factors such as the patient's general health profile, the severity and course of the patient's disorder or disposition thereto, and the judgment of the treating physician which includes interaction potentials with other needed medication.

For example, the extracts of the present invention can be administered the same way as other valerian extracts and pharmaceutical compositions thereof. Preferably, they are administered orally, more preferably as solid or liquid dosage forms.

In the following the present invention is illustrated by way of examples which relate to preferred embodiments of the invention.

### Figures

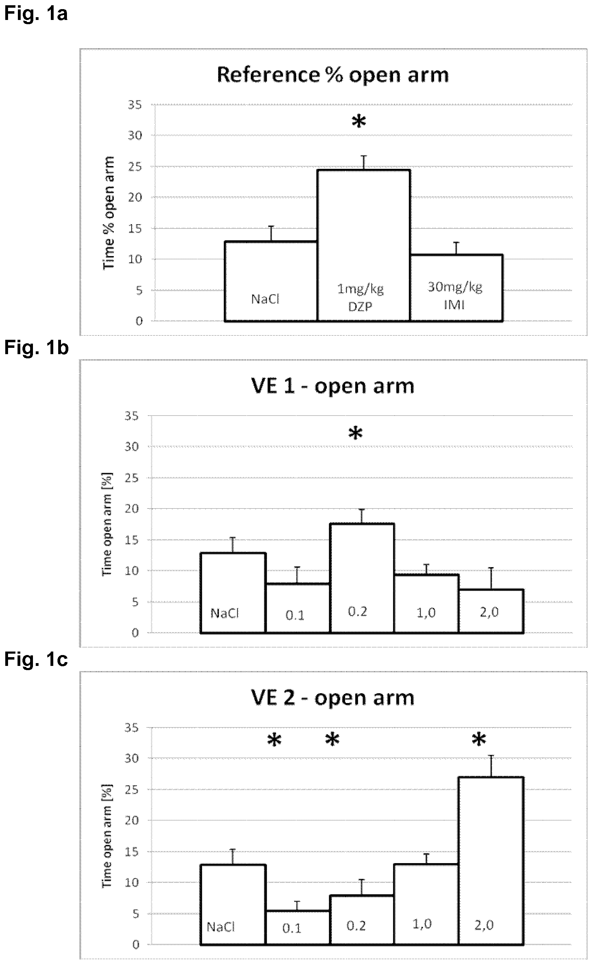

**Figs. 1a, b and c** are column graphs showing results from the elevated plus maze test for the reference substances (Fig. 1a, upper graph) and both of the valerian extracts (VE 1, Fig. 1b, middle graph; and VE 2, Fig. 1c, lower graph). On the y-coordinate the time in % is depicted which the animals spend on the open arm. Fig. 1a shows the % values for the reference substances diazepam (DZP) and imipramin (IMI) in comparison to NaCl (0.9 saline) as control. Fig. 1b presents the % results obtained for VE 1 and Fig. 1c presents the % results for VE 2. The numeric values indicated for VE 1 and 2 in Figs. 1b and 1c, respectively, refer to the sum of the valerenic acids VA and AVA administered per kg body weight.

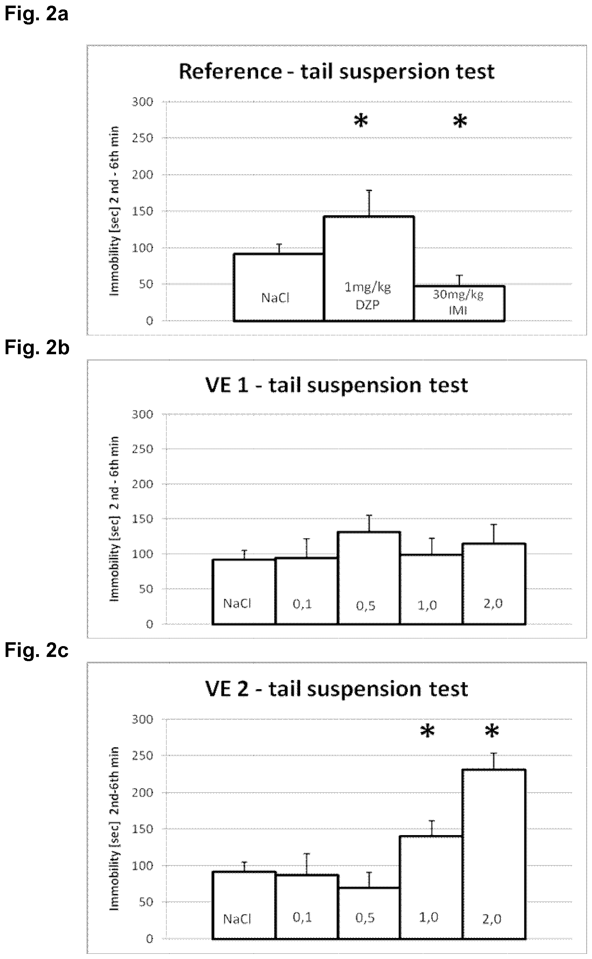

**Figs. 2a, b and c** **are** column graphs showing results from the tail suspension test for the reference substances (Fig. 2a, upper graph) and both of the valerian extracts (VE 1, Fig. 2b, middle graph; and VE 2, Fig. 2c, lower graph). On the y-coordinate the time of the immobility is depicted. Fig. 2a shows the time values for the reference sub stances diazepam (DZP) and imipramin (IMI) in comparison to NaCl as the control. Fig. 2b presents the results obtained for VE 1 and Fig. 2c shows the data for VE 2. The numeric values indicated for VE 1 and 2, respectively, refer to the sum of the valerenic acids VA and AVA administered per kg body weight.

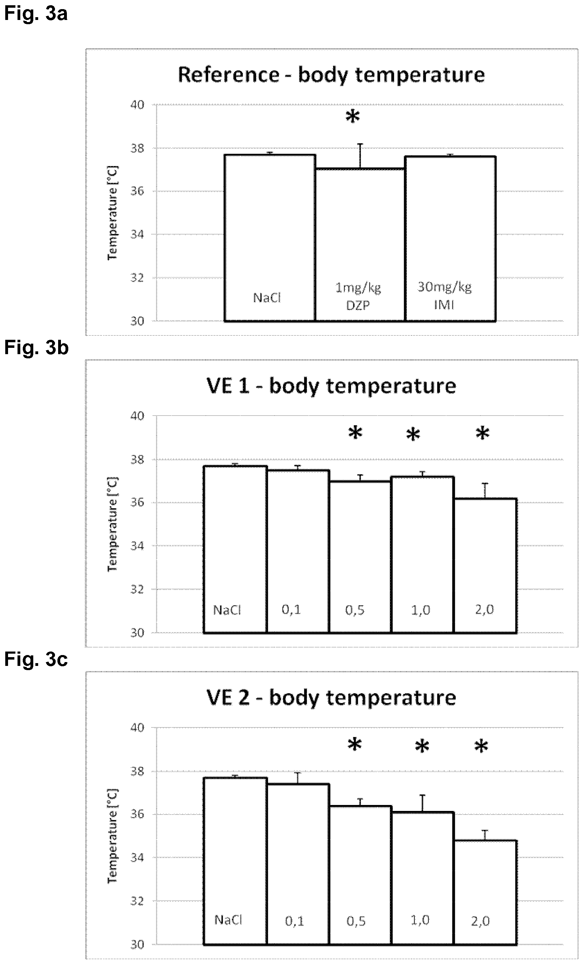

**Figs. 3a, b and c** are column graphs showing results from the measurement of the core body temperature for the reference substances (Fig. 2a, upper graph) and both of the valerian extracts (VE 1, Fig. 3b, middle graph; and VE 2, Fig. 3c, lower graph). On the y-coordinate the core body temperatures are depicted. Fig. 3a presents the temperature values for the reference substances diazepam (DZP) and imipramin (IMI) in comparison to NaCl as control. Fig. 3b presents the results obtained for VE 1 and Fig. 3c shows the data for VE 2. The numeric values indicated for VE 1 and 2, respectively, refer to the sum of the valerenic acids VA and AVA administered per kg body weight.

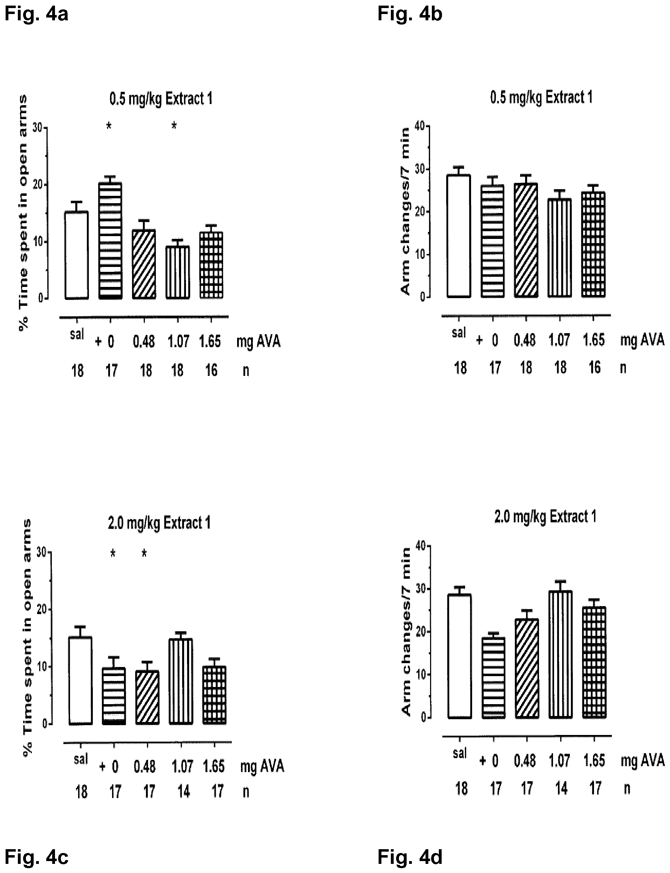

**Figs. 4a, b, c and d** are column graphs showing the results from the elevated plus maze verification test of example 5 for the valerian extract 1 (VE 1). On the y-coordinate the time in % is depicted which the animals spend on the open arm (upper and lower graphs on the left side, 4a, 4c) as well as the number of arm changes during the 7 minute observation period (upper and lower graphs on the right side, 4b, 4d). The amount of added acetoxy valerenic acid to VE-1 is indicated (mg AVA), n equals the number of animals used.

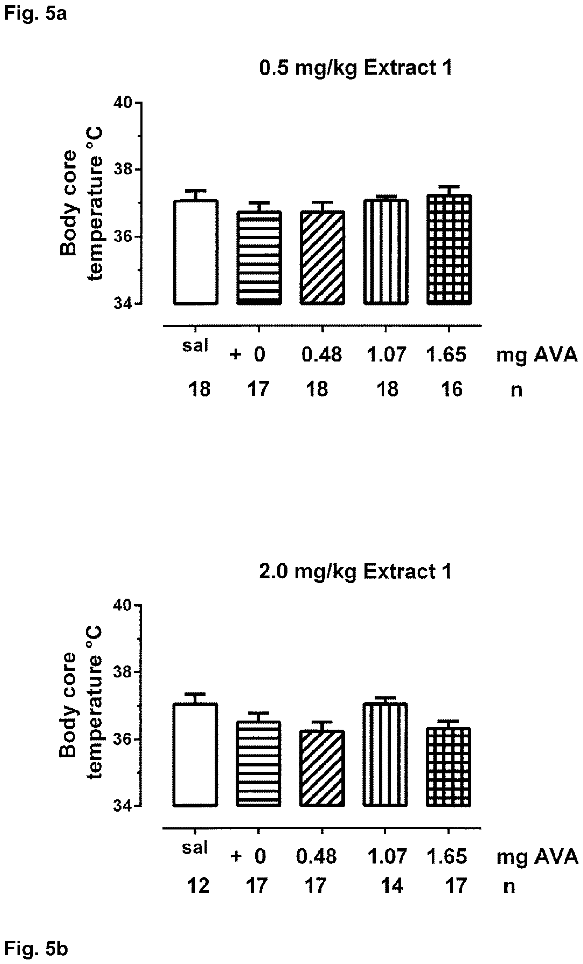

**Figs. 5a** **and b** are column graphs showing the body core temperature of the animals during the EPM verification experiment 5 for the different valerian extracts. The amount of added acetoxy valerenic acid to VE-1 is indicated (mg AVA), n equals the number of animals used.
In the figures, * indicates a significant deviance of p < 0.05 for the column below.

### Examples

### Animals

For the behavioural experiments male CD-1 mice (Charles River, Sulzfeld, Germany) were kept under controlled laboratory conditions with a light/dark cycle of 12:12 (lights on at 06.00 a.m.), temperature 20 ± 2°C, and air humidity between 55 and 60%. The animals had free access to commercial pellets (ssniff R/M-H, ssniff Spezialdiäten GmbH, Soest, Germany) and tap water. The animals were housed in groups of 10 in Macrolon III cages. After arrival, the animals were given a period of 2 weeks for habituation. At the beginning of the experiments the mice were 8 weeks old. The number of animals per group was between 12 and 18.

For the binding experiment male RjHan:WI rats (Janvier, Le Genest-Saint-Isle, France) were used. The rats were kept under controlled laboratory conditions as described above. The animals were housed in groups of 5 in Macrolon IV cages.

The work reported here was conducted in accordance with EC regulations and the National Act on the Use of Experimental Animals (Germany). The protocol was approved by the Saxony-Anhalt Committee on Animal Care.

### Example 1 - Preparation and analysis of valerian extracts (VE)

For testing the effect of different ratios of VA:AVA on GABA_{A}-related *in vivo* models two different valerian extracts (VE 1 & VE 2) were prepared as follows. Valerian roots and rhizomes from the selected strain as well as standard reference material (European Pharmacopoeia, Supplement 5.7. *Valerian* dry hydroalcoholic extract (1898)) from Poland were used. The selected strain was selected from naturally occurring strains in the field by routine analysis of the ratio of VA:AVA. It was found that naturally occurring valerian strains featuring a ratio of VA:AVA of more than 2:1 are more abundant than originally expected. The selected species was also deposited in the Herbarium (herb collection) of the Institute for Biology of the Martin-Luther-University in Halle, Germany, for publication purposes (deposition number HAL 115562). Subsequent propagation provided the necessary amount of starting material. The starting material was stirred with hydro-alcohol, i.e. hydro-ethanol (70% V/V) in a ratio of 1:5 at 45°C for 3½ days and than separated by filtration with a pressure of 2 bar. The obtained tinctures were concentrated by means of a rotary evaporator. Probes of both extracts were analyzed by Ultra Performance Liquid Chromatography (*UPLC*) (method is based on Ph Eur-Monography Valerian dry hydroalcoholic extract [6.8./1898]). The exact content of VA and AVA, the sum of both VA and AVA as well as the ratio VA: AVA are listed in the table below.

**Table 1**

| Designation | VA content (mg/l) | AVA content (mg/l) | VA + AVA (mg/l) | Ratio VA:AVA |
|---|---|---|---|---|
| Valerian extract 1 | 1165.5 | 98.3 | 1263.7 | 1 : 0.084 |
| Valerian extract 2 | 199.5 | 308.8 | 508.3 | 1 : 1.548 |

Valerian extract 1 had a VA:AVA ratio of about 12:1 and valerian extract 2 had a ratio of about 1:1.5. The content of HVA was too low to be measured quantitatively.

Valerian extracts (VE1 and VE 2), diazepam (Faustan®, AWD pharma GmbH & Co KG, Dresden, Germany) and imipramine (Sigma, Düsseldorf, Germany) as reference substances were solved in saline and administered orally using a mouse gavage needle. For control the solvent (0.9% saline) was given. Application volume was 0.1 ml/10 g body weight.

The individual and total content of VA and AVA as well as the dosage administered to the experimental animals is listed in the table below.

**Table 2**

| | [Valerian extract 1] | | | [Valerian extract 2] | | |
|---|---|---|---|---|---|---|
| VA + AVA (mg) | VA | AVA | Dosage (ml) p.o. | VA | AVA | Dosage (ml) p.o. |
| 0.1 | 0.0922 | 0.00776 | 0.16 | 0.03925 | 0.06075 | 0.20 |
| 0.5 | 0.4610 | 0.0388 | 0.40 | 0.19630 | 0.30380 | 0.98 |
| 1.0 | 0.9220 | 0.0776 | 0.79 | 0.39250 | 0.60750 | 1.97 |
| 2.0 | 1.8440 | 0.1522 | 1.58 | 0.78500 | 1.21500 | 3.93 |

| | | | | | | |
|---|---|---|---|---|---|---|
| VA: valerenic acid; AVA: acetoxyvalerenic acid; HVA: hydroxyvalerenic acid [lower than level of quantification] | | | | | | |

### Example 2 - Elevated plus maze test (EPM)

Situational anxiety was measured in the elevated plus maze test. The maze was made of black polyvinyl chloride and had two open and two closed arms (50 x 10 x 40 cm) mounted 50 cm above the floor. The floor of the arms was smooth. Light level was 30 lux. A mouse was placed in the central platform of the apparatus facing a closed arm. A camera on the ceiling of the test room was used to score and tape the animals' behaviour from an adjacent room for a period of 7 min. The number of entries into open arms, time spent on open arms and time spent on closed arms were recorded. To evaluate the anxiolytic effect, the time spent on the open arm was determined, which is prolonged by anxiolytic substances. In addition, total changes in movements were also analyzed, which reflect locomotor activity. (Rodgers RJ. Animal models of anxiety: where next? Behav Pharmacol 1997;8:477-96). The maze was cleaned after each trial.

### Example 3 - Tail suspension test

The anti-depressant-like effect of valerian extract was measured using the tail suspension test (L. Steru, R. Chermat, B. Thierry and P. Simon, The tail suspension test: a new method for screening antidepressants in mice. Psychopharmacology (Berl), 85 (1985), pp. 367-370). The test was carried out immediately after measuring anxiety in the elevated plus-maze. The mice were suspended by their tails using adhesive tape placed approximately 1 cm from the tip of the tail and hung approximately 30 cm above the table. The animals were suspended for 6 min, and the duration of immobility was scored manually during the test. Immobility was defined as the absence of any limb or body movements, with the exception of those caused by respiration, when the mice hung passively and completely motionless. Immobility measured from minute 2 to minute 6 was used to evaluate drug effects.

### Example 4 - Body core temperature (BCT)

Following the tail suspension test, body core temperature was measured with a digital thermometer (ama digit) manufactured by Amarell GmbH (Kreuzwertheim, Germany). For that purpose the lubricated probe (Ø 1 mm) was gently inserted 3 cm into the rectum. The rectal temperature allows differentiation between results related to central nervous actions of the extracts and overdosing effects (Haffner F.; Die pharmakologische Wertbestimmung des Baldrians. Münch med Wschr 7: 271-272 [1929]).

### Example 5 - Elevated plus maze (EPM) verification test

For verifying the results of the EPM test of Example 2, which indicate that the ratio between VA:AVA is important for the anxiolytic activity of VE, the extract VE-1 with the high VA content and low AVA content (12:1) was again used to replicate the anxiolytic activity of 0.5 mg of valerenic acids. Then AVA (HWI Analytik GmbH, Rülzheim, Germany) was added to the extract to change the ratios of VA:AVA from 12:1(VE-1), to 1:0.5, 1:1, and 1:1.5 (VE 1-A; VE 1-B; VE 1-C), respectively. The extract amount was adapted in such a way that the sum of VAs, i.e. VA plus AVA, was always the same. The different extract amounts with respect to their VA:AVA ratios are given in table 3 below.

**Table 3a - VA and AVA in different VE compositions and the calculated amount for oral administration**

| | **VA:AVA** | **VA mg/ml** | **AVA mg/ml** | **AVA base + AVA supply (mg/ml)** | | **total VA + AVA (mg/ml)** |
|---|---|---|---|---|---|---|
| VE - 1 | 12:1 | 1.165 | 0.0983 | | | 1.2633 |
| VE 1-A | 1:0.5 | 1.165 | 0.583 | 0.0983 | 0.48 | 1.748 |
| VE 1-B | 1:1 | 1.165 | 1.165 | 0.0983 | 1.07 | 2.33 |
| VE 1-C | 1:1.5 | 1.165 | 1.75 | 0.0983 | 1.65 | 2.915 |

**Table 3b**

| | **total VA** + **AVA (mg/ml)** | **0.5 mg/kg** | **2.0 mg/kg** |
|---|---|---|---|
| VE - 1 | 0.4998 | 0.400 ml | 1.580 ml |
| VE 1-A | 1.748 | 0.286 ml | 1.144 ml |
| VE 1-B | 2.33 | 0.215 ml | 0.858 ml |
| VE 1-C | 2.915 | 0.172 ml | 0.686 ml |

VEs (0.5 and 2.0 mg/kg, with respect to the total sum of VA and AVA) were administered orally using a mouse gavage feeding needle (24 gauge, FST, Heidelberg, Germany). As control a 0.9% saline solution was administered. The given volume was 0.1 ml/10 g body weight. Following the EPM, body core temperature (BCT) was measured with a digital thermometer (ama digit) manufactured by Amarell GmbH (Kreuzwertheim, Germany). For that purpose the lubricated probe (Ø 1 mm) was gently inserted 3 cm into the rectum.

### Example 6 - Body core temperature (BCT) verification

The BCT for the animals participating in the EPM test of example 5 remained for all experimental groups in the control range (FIG. 4), even if the values for the groups which had received 2 mg/kg were somewhat different (0.5 mg/kg extract F_{4,82} = 0.725, p = 0.725; 2.0 mg/kg extract F_{4,78} = 2.48, p = 0.051). In a few animals BCT was reduced to be below 34°C (0.5 mg/kg = 1; 2.0 mg/kg = 3). The results of these animals were disregarded for data evaluation.

### Example 7 -Binding assay

³H-GABA (SA - 1,43 TBq/mmol) and ³H-flunitrazepam (SA - 1,85 TBq/mmol) were obtained from PerkinElmer (Boston, USA), diazepam and chlordiazepoxide from AWD.pharma GmbH (Dresden, Germany). The tested VEs were identical to those extracts VE-1 and VE-2 used in the other examples.

For the ³H-GABA binding assay tissue (dissected rat frontal cortex) was homogenized in 50 mM Tris-HCI buffer pH 7.4 containing 120 mM NaCl, 5 mM KCI, 2 mM CaCl₂ and 1 mM MgCl₂; for ³**H**- flunitrazepam it was homogenized in 50 mM Tris-HCI buffer pH 7.4 containing 1 mM EGTA, 10 mM EDTA and 3 mM MgCl₂, centrifuged (50.000 g, 15 min) and washed twice with the corresponding buffer. The resulting pellet was resuspended with buffer. Aliquots of the crude membrane suspension (50 µg protein) were incubated for 45 min at 37°C with 2.5 nM ³H-GABA or 1 nM ³H-flunitrazepam at 0 - 4 °C for 60 min. Specific binding was calculated by subtracting non-specific binding - defined as that seen in the presence of 1 µM unlabelled GABA or 1 µM diazepam or chlordiazepoxide - from the total binding obtained with radioligand alone. In parallel VE-1, VE-2 and AVA were used for all binding assays. The incubation was terminated by addition of ice-cold buffer and rapid filtration through glass fibre filters. The filters were washed, dried, a scintillation cocktail was added, and radioactivity was counted using a β-counter (Beckman Coulter, Germany). The EC₅₀ values were determined by the addition of unlabelled drugs (GABA, chlordiazepoxide, diazepam, AVA, VE-1 and VE-2) over a wide concentration range (10⁻¹⁰ to 10⁻⁴ M).

Statistical evaluation of the data obtained in the elevated plus maze was carried out using the Kruskal-Wallies H-test, followed by the two-tailed Mann-Whitney U-test. Body core temperature was analyzed using ANOVA. The threshold for significance was set at p < 0.05.

Both of the tested valerian extracts did not bind to the benzodiazepine receptors at the synaptosomal membranes of the rat frontal cortex, while diazepam as well as chlordiazepoxide did (Table 4). In contrast, both of the VEs bind to the GABA receptors whereby binding affinity was more expressed with VE-1 compared to VE-2 (Table 5).

**Table 4**

| EC₅₀ values of the competition of diazepam, chlodiazepoxide, VE-1 and VE-2 at ³H-flunitrazepam labelled binding sites of crude synaptosomal membranes of rat frontal cortex | | | | |
|---|---|---|---|---|
| | Diazepam | Chlordiazepoxide | VE-1 | VE-2 |
| EC₅₀ ( µM ) | 0.40 ± 0.05 | 0.64 ± 0.08 | > 50 | > 50 |

**Table 5**

| EC₅₀ values of the competition of GABA, AVA, VE-1 and VE-2 at ³**H-GABA** labelled binding sites of crude synaptosomal membranes of rat frontal cortex (the values for the VEs refer to VA in the extracts) | | | | |
|---|---|---|---|---|
| | GABA | AVA | VE-1 | VE-2 |
| EC₅₀ ( µM ) | 1.82 ± 0.21 | - | 5.61 ± 1.68 | 10.04 ± 1.34 |

All behavioural tests were performed in the light period between 8.00 a.m. and 2.00 p.m. The animals were randomly ordered for testing.

### Summary on experiments 1 to 4

The above *in vivo* experiments demonstrate that the ratio of VA to AVA is an important parameter for GABA_{A}-active valerian extracts. Even though the total content of VA and AVA was the same for the two valerian extracts used in the experiments above, only valerian extract 1, which had a significantly higher VA content than valerian extract 2, demonstrated anxiolytic effects. Furthermore, a higher content in VA compared to AVA is not only more effective, it also allows for reducing the dosage and, thus for avoiding or reducing valerian-related intoxication. Therefore, valerian extracts of the present invention are more effective and safe at the same time.

### Summary on experiments 5 to 7

For the animal groups in the EPM verification test treated with 0.5 mg/kg extract + AVA a significant effect was found (X²_{4,87} = 24.37, p < 0.001). The control animals (treated with saline) spent 15.15 ± 1.76 % time in the open arm. Animals which had received VE-1 (total amount of VAs 0.5 mg/kg; VA:AVA = 12:1) exhibited extended times in the open arm (20.16 ± 1.24%; U_{2, 18, 17} = 89.0, p = 0.035). This is to be interpreted as an anxiolytic effect (FIG 4.). The results of the other animal groups, which had received VE with different ratios of valerenic acids (VA : AVA = 1:0.5 or 1:1.5) were either in the range of the saline control group or demonstrated just the opposite effect, i.e. an anxiogenic action (VA:AVA = 1:1; U _{2, 18, 18} = 79.5, p = 0.008).

The groups treated with 2.0 mg/kg extract + AVA also differed significantly (X²_{4,86} = 11.05, p = 0.026). VE -1 in an elevated amount (total amount of VAs 2.0 mg/kg; VA:AVA = 12:1) induced rather an anxiogenic activity (9.61 ± 2.02 %, U _{2, 18, 17} = 93.0, p = 0.049. A similar result was obtained with VE -1 A (VA:AVA = 1:0.5) (9.06 ± 1.64 %, U _{2, 18, 17} = 86.0. p = 0.027) whereas the results for both of the other VEs remained in the range as for saline.

The locomotor activity (total arm changes) was similar in all experimental groups (0.5 mg/kg extract X² = 4.89, p = 0.298; 2.0 mg/kg extract X² = 6.27, p = 0.18, Fig. 4).

The BCT remained in the control range for all experimental groups (FIG. 5), even if the values for the groups which had received 2 mg/kg were somewhat different (0.5 mg/kg extract F = 0.725, p = 0.725; 2.0 mg/kg extract F = 2.48, p = 0.051). In a few animals BCT was reduced to be below 34°C (0.5 mg/kg = 1; 2.0 mg/kg = 3). The results of these animals were disregarded for data evaluation.

Both of the tested valerian extracts did not bind to the benzodiazepine receptors at the synaptosomal membranes of the rat frontal cortex, while diazepam as well as chlordiazepoxide did. In contrast, both of the VEs bind to the GABA receptors whereby binding affinity was more expressed with VE-1 compared to VE-2.

## Claims

1. Valerian extract for use in the treatment and prophylaxis of GABA_{A}-related disorders selected from the group consisting of anxiety, depression, restlessness and insomnia, wherein the ratio of valerenic acid (VA) to acetoxyvalerenic acid (AVA) in the extract is at least 2:1 by weight.

2. The extract for use according claim 1, wherein the ratio of valerenic acid (VA) to acetoxyvalerenic acid (AVA) in the extract is at least 3:1, preferably at least 4:1, more preferably at least 10:1, most probably at least about 12:1 by weight.

3. The extract for use according to claim 1 or 2, wherein the solvent for producing the extract is selected from the group consisting of petrolether, ethyl acetate and/or aqueous alcohols (hydro-alcohols), preferably hydro-alcohols with an alcohol content between 40 - 80%, (v/v) more preferably aqueous methanol or ethanol, most preferably aqueous ethanol.

4. Pharmaceutical composition comprising a valerian extract, wherein the ratio of valerenic acid (VA) to acetoxyvalerenic acid (AVA) in the extract is at least 2:1 by weight.

5. The pharmaceutical composition according to claim 4, wherein the ratio of valerenic acid (VA) to acetoxyvalerenic acid (AVA) in the extract is at least 3:1, preferably at least 4:1, more preferably at least 10:1, most probably at least about 12:1 by weight.

6. The pharmaceutical composition according to claim 4 or 5, wherein the solvent for producing the extract is selected from the group consisting of petrolether, ethyl acetate and/or aqueous alcohols (hydro-alcohols), preferably hydro-alcohols with an alcohol content between 40 - 80%, (v/v), more preferably aqueous methanol or ethanol, most preferably aqueous ethanol.

7. The pharmaceutical composition according to any one of claims 4 to 6, further comprising at least one extract selected from the group of extracts produced from balm [melissa], hop and passion flower, preferably further comprising extract(s) produced from balm [melissa], hop and passion flower.

8. Method for preparing a valerian extract comprising the steps of:
a) selecting valerian roots and/or rhizomes, wherein the ratio of valerenic acid (VA) to acetoxyvalerenic acid (AVA) is at least 2:1 by weight,
b) extracting the valerenic acid (VA) and acetoxyvalerenic acid (AVA) from the roots and/or rhizomes using a suitable solvent,
c) optionally further processing the raw extract to make it suitable for human administration.

9. Method for preparing a valerian extract comprising the steps of:
a) providing valerian roots and/or rhizomes,
b) extracting the valerenic acid (VA) and acetoxyvalerenic acid (AVA) from the roots and/or rhizomes using a suitable solvent,
c) adapting the content of valerenic acid (VA) and acetoxyvalerenic acid (AVA) in the extract so that the ratio of valerenic acid (VA) to acetoxyvalerenic acid (AVA) is at least 2:1 by weight,
d) optionally further processing the extract to make it suitable for human administration.

10. The method according to claim 8 or 9, wherein the content of valerenic acid (VA) and acetoxyvalerenic acid (AVA) in the extract is adapted so that the ratio of valerenic acid (VA) to acetoxyvalerenic acid (AVA) is at least 3:1, preferably at least 4:1, more preferably at least 10:1, most probably at least about 12:1 by weight.

11. The method according to claim 8 or 10, wherein the solvent for producing the extract is selected from the group consisting of petrolether, ethyl acetate and/or aqueous alcohols (hydro-alcohols), preferably hydro-alcohols with an alcohol content between 40 - 80%, (v/v), more preferably aqueous methanol or ethanol, most preferably aqueous ethanol.

## Patentansprüche

1. Baldrianextrakt zur Verwendung in der Behandlung und Vorbeugung von GABA_{A}-bezogenen Erkrankungen ausgewählt aus der Gruppe bestehend aus Angstzustand, Depression, Ruhelosigkeit und Schlaflosigkeit, wobei das Verhältnis von Valerensäure (VS) zu Acetoxyvalerensäure (AVS) in dem Extrakt wenigstens 2:1 nach Gewicht ist.

2. Der Extrakt zur Verwendung gemäss Anspruch 1, wobei das Verhältnis von Valerensäure (VS) zu Acetoxyvalerensäure (AVS) in dem Extrakt wenigstens 3:1, vorzugsweise wenigstens 4:1, mehr bevorzugt wenigstens 10:1, am meisten bevorzugt wenigstens ungefähr 12:1 nach Gewicht ist.

3. Der Extrakt zur Verwendung gemäss Anspruch 1 oder 2, wobei das Lösungsmittel zur Herstellung des Extrakts ausgewählt ist aus der Gruppe bestehend aus Petrolether, Ethylacetat und/oder wässrigen Alkoholen (Hydroalkoholen), vorzugsweise Hydroalkoholen mit einem Alkoholgehalt zwischen 40 - 80 % (v/v), mehr bevorzugt wässriger Methanol oder Ethanol, am meisten bevorzugt wässriger Ethanol.

4. Pharmazeutische Zusammensetzung umfassend einen Baldrianextrakt, wobei das Verhältnis von Valerensäure (VS) zu Acetoxyvalerensäure (AVS) in dem Extrakt wenigstens 2:1 nach Gewicht ist.

5. Die pharmazeutische Zusammensetzung gemäss Anspruch 4, wobei das Verhältnis von Valerensäure (VS) zu Acetoxyvalerensäure (AVS) in dem Extrakt wenigstens 3:1, vorzugsweise wenigstens 4:1, mehr bevorzugt wenigstens 10:1, am meisten bevorzugt wenigstens ungefähr 12:1 nach Gewicht ist.

6. Die pharmazeutische Zusammensetzung gemäss Anspruch 4 oder 5, wobei das Lösungsmittel zur Herstellung des Extrakts ausgewählt ist aus der Gruppe bestehend aus Petrolether, Ethylacetat und/oder wässrigen Alkoholen (Hydroalkoholen), vorzugsweise Hydroalkoholen mit einem Alkoholgehalt zwischen 40 - 80 % (v/v), mehr bevorzugt wässriger Methanol oder Ethanol, am meisten bevorzugt wässriger Ethanol.

7. Die pharmazeutische Zusammensetzung gemäss einem der Ansprüche 4 bis 6, zusätzlich umfassend wenigstens einen Extrakt ausgewählt aus der Gruppe von Extrakten, die aus Melisse, Hopfen und Passionsblume hergestellt werden, vorzugsweise zusätzlich umfassend Extrakt(e) aus Melisse, Hopfen und Passionsblume hergestellte Extrakt(e).

8. Verfahren zur Herstellung eines Baldrianextrakts umfassend die Schritte:
a) Auswählen von Baldrianwurzeln und/oder -rhizomen, wobei das Verhältnis von Valerensäure (VS) zu Acetoxyvalerensäure (AVS) wenigstens 2:1 nach Gewicht ist,
b) Extrahieren der Valerensäure (VS) und Acetoxyvalerensäure (AVS) aus den Wurzeln und/oder Rhizomen unter Verwendung eines geeigneten Lösungsmittels,
c) wahlweise Weiterverarbeitung des Rohextrakts, um diesen für die Verabreichung am Menschen geeignet zu machen.

9. Verfahren zur Herstellung eines Baldrianextrakts umfassend die Schritte:
a) Bereitstellen von Baldrianwurzeln und/oder -rhizomen,
b) Extrahieren der Valerensäure (VS) und Acetoxyvalerensäure (AVS) aus den Wurzeln und/oder Rhizomen unter Verwendung eines geeigneten Lösungsmittels,
c) Anpassung des Gehalts an Valerensäure (VA) und Acetoxyvalerensäure (AVS) in dem Extrakt, so dass das Verhältnis von Valerensäure (VS) zu Acetoxyvalerensäure (AVS) wenigstens 2:1 nach Gewicht ist,
d) wahlweise Weiterverarbeitung des Rohextrakts, um diesen für die Verabreichung am Menschen geeignet zu machen.

10. Das Verfahren gemäss Anspruch 8 oder 9, wobei der Gehalt der Valerensäure (VS) und Acetoxyvalerensäure (AVS) in dem Extrakt so angepasst wird, dass das Verhältnis von Valerensäure (VS) zu Acetoxyvalerensäure (AVS) wenigstens 3:1, vorzugsweise wenigstens 4:1, mehr bevorzugt wenigstens 10:1, am meisten bevorzugt wenigstens ungefähr 12:1 nach Gewicht ist.

11. Das Verfahren gemäss Anspruch 8 oder 10, wobei das Lösungsmittel zur Herstellung des Extrakts ausgewählt ist aus der Gruppe bestehend aus Petrolether, Ethylacetat und/oder wässrigen Alkoholen (Hydroalkoholen), vorzugsweise Hydroalkoholen mit einem Alkoholgehalt zwischen 40 - 80 % (v/v), mehr bevorzugt wässriger Methanol oder Ethanol, am meisten bevorzugt wässriger Ethanol.

## Revendications

1. Extrait de valériane pour utilisation dans le traitement et la prophylaxie des troubles liés au GABA_{A} sélectionnés dans le groupe constitué par l'anxiété, la dépression, l'agitation et l'insomnie, où le rapport de l'acide valérénique (VA) à l'acide acétoxyvalérénique (AVA) dans l'extrait est d'au moins 2:1 en poids.

2. Extrait pour utilisation selon la revendication 1, où le rapport de l'acide valérénique (VA) à l'acide acétoxyvalérénique (AVA) dans l'extrait est d'au moins 3:1, de préférence d'au moins 4:1, plus préférentiellement d'au moins 10:1, le plus probablement d'au moins environ 12:1 en poids.

3. Extrait pour utilisation selon la revendication 1 ou 2, où le solvant pour produire l'extrait est sélectionné dans le groupe constitué par l'éther de pétrole, l'acétate d'éthyle et/ou les alcools aqueux (hydro-alcools), de préférence les hydro-alcools ayant une teneur en alcool entre 40 et 80% (v/v), plus préférentiellement le méthanol aqueux ou l'éthanol aqueux, le plus préférentiellement l'éthanol aqueux.

4. Composition pharmaceutique comprenant un extrait de valériane, où le rapport de l'acide valérénique (VA) à l'acide acétoxyvalérénique (AVA) dans l'extrait est d'au moins 2:1 en poids.

5. Composition pharmaceutique selon la revendication 4, où le rapport de l'acide valérénique (VA) à l'acide acétoxyvalérénique (AVA) dans l'extrait est d'au moins 3:1, de préférence d'au moins 4:1, plus préférentiellement d'au moins 10:1, le plus probablement d'au moins environ 12:1 en poids.

6. Composition pharmaceutique selon la revendication 4 ou 5, où le solvant pour produire l'extrait est sélectionné dans le groupe constitué par l'éther de pétrole, l'acétate d'éthyle et/ou les alcools aqueux (hydro-alcools), de préférence les hydro-alcools ayant une teneur en alcool entre 40 et 80% (v/v), plus préférentiellement le méthanol aqueux ou l'éthanol aqueux, le plus préférentiellement l'éthanol aqueux.

7. Composition pharmaceutique selon l'une quelconque des revendications 4 à 6, comprenant en outre au moins un extrait sélectionné dans le groupe d'extraits produits à partir de mélisse citronnelle [mélisse], de houblon et de passiflore, de préférence comprenant en outre un/des extrait(s) produit(s) à partir de mélisse citronnelle [mélisse], de houblon et de passiflore.

8. Procédé de préparation d'un extrait de valériane comprenant les étapes de:
a) sélection de racines et/ou de rhizomes de valériane, où le rapport de l'acide valérénique (VA) à l'acide acétoxyvalérénique (AVA) est d'au moins 2:1 en poids,
b) extraction de l'acide valérénique (VA) et de l'acide acétoxyvalérénique (AVA) à partir des racines et/ou des rhizomes à l'aide d'un solvant approprié,
c) facultativement, traitement supplémentaire de l'extrait brut pour le rendre adapté à l'administration à un humain.

9. Procédé de préparation d'un extrait de valériane comprenant les étapes de:
a) fourniture de racines et/ou de rhizomes de valériane,
b) extraction de l'acide valérénique (VA) et de l'acide acétoxyvalérénique (AVA) à partir des racines et/ou des rhizomes à l'aide d'un solvant approprié,
c) adaptation de la teneur en acide valérénique (VA) et en acide acétoxyvalérénique (AVA) dans l'extrait de sorte que le rapport de l'acide valérénique (VA) à l'acide acétoxyvalérénique (AVA) soit d'au moins 2:1 en poids,
d) facultativement, traitement supplémentaire de l'extrait pour le rendre adapté à l'administration à un humain.

10. Procédé selon la revendication 8 ou 9, où la teneur en acide valérénique (VA) et en acide acétoxyvalérénique (AVA) dans l'extrait est adaptée de sorte que le rapport de l'acide valérénique (VA) à l'acide acétoxyvalérénique (AVA) soit d'au moins 3:1, de préférence d'au moins 4:1, plus préférentiellement d'au moins 10:1, le plus probablement d'au moins environ 12:1 en poids.

11. Procédé selon la revendication 8 ou 10, où le solvant pour produire l'extrait est sélectionné dans le groupe constitué par l'éther de pétrole, l'acétate d'éthyle et/ou les alcools aqueux (hydro-alcools), de préférence les hydro-alcools ayant une teneur en alcool entre 40 et 80% (v/v), plus préférentiellement le méthanol aqueux ou l'éthanol aqueux, le plus préférentiellement l'éthanol aqueux.
